# EUROPÄISCHE PATENTSCHRIFT

(11) **EP 4 801 391 B1**
(45) Veröffentlichungstag und Bekanntmachung des Hinweises auf die Patenterteilung: **14.10.2026**
(21) Anmeldenummer: 25835409.1
(22) Anmeldetag: 12.12.2025
(51) Int. Cl.: A61B 17/70, A61B 17/80, A61B 17/88

(54) **IMPLANTAT ZUR VERBINDUNG BENACHBARTER WIRBEL UND MEDIZINISCHES SYSTEM**
IMPLANT FOR CONNECTING ADJACENT VERTEBRAE, AND MEDICAL SYSTEM
IMPLANT POUR RELIER DES VERTÈBRES ADJACENTES ET SYSTÈME MÉDICAL

(30) Priorität: 18.12.2024 DE 102024138480
(43) Veröffentlichungstag der Anmeldung: 09.09.2026
(73) Patentinhaber: AESCULAP AG, 78532 Tuttlingen (DE)
(72) Erfinder: STERN, Andreas, 78087 Mönchweiler (DE)
(74) Vertreter: Patentanwälte Ruff, Wilhelm, Beier, Dauster & Partner mbB
(86) Internationale Anmeldenummer: PCT/EP2025/086774
(87) Internationale Veröffentlichungsnummer: WO 2026/131496

(56) Entgegenhaltungen:
- WO-A1-2023/079508
- US-A1- 2012 158 068
- US-A1- 2018 344 362
- US-A1- 2021 378 722

## Beschreibung

Die Erfindung betrifft ein Implantat zur Verbindung benachbarter Wirbel sowie ein medizinisches System aufweisend ein solches Implantat und ein Biegewerkzeug.

Wirbelsäulenimplantate werden dazu verwendet, mehrere Wirbel miteinander zu verbinden und die Wirbelsäule zu stabilisieren. Derartige Implantate werden insbesondere im Halswirbelbereich verwendet und dann als zervikale Platte bezeichnet. Das Implantat wird mittels Befestigungsschrauben mit den Wirbeln verbunden. Es gibt dynamische Implantate, die eine Rotation und Translation der in entsprechenden Aufnahmen des Implantats aufgenommenen Befestigungsschrauben zulassen. Dadurch können sich die verbundenen Wirbel auch bei eingesetztem Implantat relativ zueinander bewegen, wobei sie gleichzeitig durch das Implantat gestützt sind. Vor der Befestigung des Implantats an den Wirbeln wird das Implantat individuell an die Wirbelsäule des Patienten angepasst. Hierzu wird das Implantat mittels eines Biegewerkzeugs während der Operation (intraoperativ) plastisch deformiert. Ein solches Biegewerkzeug weist für gewöhnlich als Wirkabschnitte eine Biegeform und zwei Gegenhaltefortsätze auf. Um die Biegeform herum wird das Implantat gebogen. Die Gegenhaltefortsätze halten das Implantat fest und werden um die Biegeform herum bewegt. Dabei nehmen die Gegenhaltefortsätze einen Teil des Implantats mit, so dass das Implantat zwischen den Gegenhaltefortsätzen und der Biegeform gebogen wird.

Ein seitens der Anmelderin am Markt angebotenes Implantat ist unter der Bezeichnung zervikales Plattensystem ABC2 bekannt. Das bekannte Plattensystem umfasst ein plattenförmiges Implantat und mehrere Befestigungsschrauben, die in zugeordneten Durchgangsöffnungen in dem Implantat aufgenommen und in den zu verbindenden Wirbeln verschraubt sind. Zum formmäßigen Anpassen des Implantats an die Bedürfnisse des Patienten weist das Implantat auf der von den Wirbeln abgewandten Seite eine Nut auf, so dass an dieser Stelle das Material des Implantats geschwächt ist und eine Verformung im Wesentlichen im Bereich der Nut erfolgt.

Aus der US 2021/0378722 A1 ist ein Implantat in Form einer Platte zur Fixierung von Osteotomien und Gelenkversteifungen bekannt. Die Platte lässt sich mittels eines Biegewerkzeugs intraoperativ verbiegen. Die Platte weist eine Nut auf der Seite auf, die im an Knochen befestigten Zustand den Knochen zugewandt ist. Gegenhaltefortsätze des Biegewerkzeugs werden in der Nut angeordnet. Die Nut ermöglicht ein einfaches Entfernen des Biegewerkzeugs nach dem Befestigen der Platte an den Knochen. Randseitige Begrenzungen der Nut dienen als Zentrierungshilfen für das Biegewerkzeug. Die Platte hat in einer Ruhestellung eine längsgekrümmte Form. Während der Operation wird die Platte mittels des Biegewerkzeugs elastisch verformt, sodass sie gerade längserstreckt ist, und wird im gestreckten Zustand mit den Knochen verbunden. Bei Verringerung der auf die Platte ausgeübten Biegekraft kehrt die Platte selbstständig in ihre gekrümmte Ausgangsstellung zurück. Die Platte ist aus einem elastischen Material hergestellt, um die beschriebene Streckung und selbständige Rückkehr in den gekrümmten Zustand zu ermöglichen.

Aufgabe der Erfindung ist es, ein Implantat zur Verbindung benachbarter Wirbel bereitzustellen, das einen gegenüber dem Stand der Technik verbesserten Aufbau aufweist und eine einfache Anwendung erlaubt.

Diese Aufgabe wird durch das Bereitstellen eines Implantats mit den Merkmalen des Anspruchs 1 und eines medizinischen Systems mit den Merkmalen des Anspruchs 8 gelöst.

Das erfindungsgemäße Implantat weist eine Vorderseite und eine Rückseite auf, die der Vorderseite in Dickenrichtung des Implantats gegenüber liegt. Das Implantat weist zudem wenigstens zwei Durchgangsöffnungen auf, die jeweils zwischen der Vorderseite und der Rückseite längserstreckt und zur Aufnahme einer Befestigungsschraube eingerichtet sind. Das Implantat umfasst wenigstens einen Biegeabschnitt, der unter Einwirkung eines Biegewerkzeugs auf das Implantat plastisch deformierbar ist, um eine entlang einer Längsachse des Implantats erstreckte Längskrümmung des Implantats intraoperativ anzupassen. Der Biegeabschnitt weist wenigstens eine Werkzeugaufnahmenut auf, die in Dickenrichtung in die Vorderseite oder die Rückseite eingesenkt und entlang einer Querachse des Implantats längserstreckt ist. Die wenigstens eine Werkzeugaufnahmenut ist zur Aufnahme eines Wirkabschnitts des Biegewerkzeugs eingerichtet. Die Werkzeugaufnahmenut erstreckt sich nur über einen Teil der Breite des Implantats. Das insbesondere als Wirbelsäulenimplantat ausgestaltete Implantat ist zur Verbindung von zwei, drei oder mehr Wirbeln vorgesehen, bei denen es sich insbesondere um Halswirbel handeln kann. Die Rückseite des Implantats ist den Wirbeln im implantierten Zustand zugewandt. Die Vorderseite weist in Blickrichtung des Patienten. Jeweils wenigstens eine der Durchgangsöffnungen ist einem der zu verbindenden Wirbel zugeordnet, so dass die in den Durchgangsöffnungen aufgenommenen Befestigungsschrauben die mehreren Wirbel über das Implantat miteinander verbinden.

Zwecks Anpassung der besagten Längskrümmung greift das Biegewerkzeug in dem Biegeabschnitt an, so dass eine Deformation des Implantats im Wesentlichen lokal im Bereich des Biegeabschnitts erfolgt. Im Speziellen ist im Gebrauch ein Wirkabschnitt des Biegewerkzeugs, also beispielsweise die erwähnte Biegeform oder einer der Gegenhaltehaltefortsätze, in der Werkzeugaufnahmenut aufgenommen. Zudem kann vorteilhaft eines oder mehrere Fenster in der zervikalen Platte als Mittel zur Ausrichtung des Biegewerkzeugs relativ zum Biegeabschnitt dienen, indem ein Abschnitt des Biegewerkzeugs in das Fenster eintaucht.

Das Anpassen der Längskrümmung des Implantats erfolgt während der Operation (intraoperativ), wodurch das Implantat individuell auf eine Kontur der Wirbel, das heißt einen Verlauf der Wirbelsäule im Bereich der zu verbindenden Wirbel, angepasst werden kann. Falls mehrere Biegeabschnitte entlang der Längsachse des Implantats vorgesehen sind, kann das Implantat im Bereich jedes der Biegeabschnitte unabhängig von den anderen Biegeabschnitten deformiert werden. Dadurch kann das Implantat erforderlichenfalls mit einer entlang der Längsachse komplex geschwungenen Form versehen werden, die beispielsweise an die Kontur von drei oder mehr Wirbel angepasst sein kann.

Die Werkzeugaufnahmenut verläuft quer zur Längsachse entlang des Biegeabschnitts, so dass durch dessen Deformation der Krümmungsradius in Richtung der Längsachse verändert wird. Im Bereich der Werkzeugaufnahmenut ist in Dickenrichtung des Implantats das Material geschwächt. Dadurch erfolgt eine Deformation des Implantats zunächst und im Wesentlichen im Bereich der Werkzeugaufnahmenut. Durch eine konstruktive Anpassung der Tiefe der Werkzeugaufnahmenut und dadurch der verbleibenden Stärke des Materials in Dickenrichtung im Bereich der Werkzeugaufnahmenut lässt sich eine gewünschte Biegesteifigkeit des Implantats erreichen. Die Längsachse, Querachse und Dickenrichtung sind jeweils senkrecht zueinander orientiert.

Vorzugsweise ist die Werkzeugaufnahmenut konkav ausgestaltet und das Biegewerkzeug greift mit einem konvexen Wirkabschnitt in die Werkzeugaufnahmenut ein. Besonders bevorzugt sind die Werkzeugaufnahmenut und der Wirkabschnitt des Biegewerkzeugs komplementär zueinander ausgebildet, so dass eine möglichst große Auflagefläche und/oder Kontaktfläche zwischen dem Implantat und dem Biegewerkzeug erreicht wird. Bei bevorzugten Ausführungsformen erfolgt die Aufnahme des Wirkabschnitts in der Werkzeugaufnahmenut formschlüssig, insbesondere entlang der Längsachse.

Durch die Verringerung des zur Deformation erforderlichen Kraftaufwands und die Vergrößerung der Auflagefläche wird die Gefahr einer unbeabsichtigten Deformation des Implantats aufgrund des Kontakts mit dem Biegewerkzeug im Wirkabschnitt, beispielsweise in Form von Druckstellen, verringert oder vermieden.

Die Werkzeugaufnahmenut erstreckt sich nicht über die gesamte Breite des Implantats entlang der Querachse. Hierdurch kann das Implantat entlang der Querachse im an die Werkzeugaufnahmenut angrenzenden Bereich beliebig ausgestaltet sein, ohne dass die Werkzeugaufnahmenut oder der weitere Aufbau des Implantats in Konflikt geraten, beispielsweise mit einer Durchgangsöffnung oder einem auf der Vorderseite angeordneten Verriegelungsmechanismus.

Bevorzugt umfasst das Implantat auf den entlang der Querachse gegenüberliegenden Seite des Implantats jeweils eine Werkzeugaufnahmenut, die spiegelbildlich zueinander ausgestaltet sind. Mit anderen Worten setzt sich die Werkzeugaufnahmenut entlang der Querachse auf der gegenüberliegenden Seite des Implantats fort, nachdem sie in einem Mittenbereich unterbrochen worden ist. Bei dieser Ausführungsform kann ein Biegewerkzeug im Gebrauch auf beiden Seiten auf das Implantat einwirken, ohne dies über die gesamte Breite des Implantats tun zu müssen.

In Ausgestaltung der Erfindung umfasst der Biegeabschnitt eine vordere Werkzeugaufnahmenut auf der Vorderseite und eine hintere Werkzeugaufnahmenut auf der Rückseite, die einander in Bezug auf die Längsachse zumindest teilweise überlappen. Mit "Überlappen" ist gemeint, dass die vordere Werkzeugaufnahmenut und die hintere Werkzeugaufnahmenut in Bezug auf die Längsachse zumindest teilweise auf derselben Höhe vorgesehen sind. Durch die beiden Werkzeugaufnahmenuten ist der Querschnitt des Implantats von beiden Seiten des Implantats verringert, was das Deformationsverhalten und die Stabilität des Implantats begünstigen kann. Zudem kann durch diese Ausgestaltung ein gleichmäßigeres Biegen in beide Richtungen ermöglichen, also in Richtung der Vorderseite oder der Rückseite. Vorteilhaft dient die eine Werkzeugaufnahmenut der Aufnahme der Biegeform und die andere Werkzeugaufnahmenut der Aufnahme eines der beiden oder beider Gegenhaltefortsätze des Biegewerkzeugs. Besonders bevorzugt sind die vordere und die hintere Werkzeugaufnahmenut in Bezug auf die Dickenrichtung spiegelbildlich und/oder symmetrisch ausgebildet.

In weiterer Ausgestaltung der Erfindung umfasst die Werkzeugaufnahmenut einen Nutgrund mit wenigstens einer ebenen Fläche. Hierdurch ist eine stets gleichmäßige Auflage des Wirkabschnitts des Biegewerkzeugs gegen das Implantat mit einer vergleichsweise großen Auflagefläche sichergestellt. Die Anlage des Wirkabschnitts gegen den Nutgrund ist stets gleichbleibend, unabhängig von einer relativen Positionierung, was den Gebrauch erleichtert. Der Nutgrund besteht bei vorteilhaften Ausführungsformen aus einer einzigen Fläche oder aus mehreren parallelen oder zueinander geneigten ebenen Flächen.

In weiterer Ausgestaltung der Erfindung ist die Werkzeugaufnahmenut mehrstufig, so dass die Werkzeugaufnahmenut einen ersten Nutabschnitt mit einer ersten Nuttiefe und einen zweiten Nutabschnitt mit einer zweiten Nuttiefe umfasst, wobei die erste Nuttiefe geringer als die zweite Nuttiefe ist. Aufgrund des dort geringeren Querschnitts erfolgt die Deformation überwiegend im Bereich des zweiten Nutabschnitts. Gleichzeitig dienen der erste und der zweite Nutabschnitt der Aufnahme der Biegeform und der Gegenhaltefortsätze und einer Ausrichtung des Biegewerkzeugs und des Implantats. Bei bevorzugten Ausführungsformen ist zu beiden Seiten des zweiten Nutabschnitts entlang der Längsachse ein erster Nutabschnitt vorgesehen. Anders ausgedrückt ist bei diesen Ausführungsformen der erste Nutabschnitt zweiteilig, so dass ein erster Teil des ersten Nutabschnitts entlang der Längsachse vor dem zweiten Nutabschnitt und ein zweiter Teil nach dem zweiten Nutabschnitt liegt.

In weiterer Ausgestaltung der Erfindung ist der zweite Nutabschnitt in Richtung der Längsachse gesehen länger als der erste Nutabschnitt. Hierdurch ist ein besonders langer Bereich entlang der Längsachse bereitgestellt, in dem überwiegend die Deformation des Implantats erfolgt. Die Biegesteifigkeit ist dadurch sehr gering gehalten. Dabei kann gleichzeitig die Länge des Biegeabschnitts insgesamt gering gehalten werden.

In weiterer Ausgestaltung der Erfindung sind der erste Nutabschnitt und der zweite Nutabschnitt miteinander durch einen Übergangsabschnitt verbunden, in dem der Nutgrund vom zweiten Nutabschnitt zum ersten Nutabschnitt ansteigt. Der Übergangsabschnitt kann zum einen der Ausrichtung des Biegewerkzeugs relativ zum Biegeabschnitt dienen und zum anderen eine weitere Anlagefläche bereitstellen, gegen die ein Wirkabschnitt des Biegewerkzeugs oder ein Teilabschnitt eines solchen Wirkabschnitts, insbesondere die Biegeform, im Gebrauch anliegt. Der Übergangsabschnitt schafft einen Übergang vom zweiten zum ersten Nutabschnitt, der sprunghaft oder stetig ansteigen kann. Bei bevorzugten Ausführungsformen umfasst der Übergangsabschnitt eine ebene Fläche, die zwischen dem zweiten und dem ersten Nutabschnitt gleichmäßig ansteigt, beispielsweise in einem Winkel von 10° bis 80°, bevorzugt von 30° bis 60°, in Bezug auf die Ausrichtung des Nutgrunds. Bei anderen bevorzugten Ausführungsformen umfasst der Übergangsabschnitt eine gekrümmte Fläche, die zwischen dem zweiten und dem ersten Nutabschnitt in einem Radius ansteigt, beispielsweise in einem Radius von 1mm bis 3mm.

In weiterer Ausgestaltung der Erfindung ist wenigstens ein Verriegelungsmechanismus zum Verriegeln der Befestigungsschrauben in den Durchgangsöffnungen vorgesehen. Der Verriegelungsmechanismus verhindert in einem verriegelten Zustand eine unbeabsichtigte Bewegung der Befestigungsschrauben aus den Durchgangsöffnungen heraus. Vorteilhaft ist der Verriegelungsmechanismus in Bezug auf die Querachse in einem Mittenbereich angeordnet.

Für das medizinisches System wird die der Erfindung zugrunde liegende Aufgabe dadurch gelöst, dass das System ein erfindungsgemäßes Implantat und ein Biegewerkzeug aufweist, das zum intraoperativen Anpassen einer Längskrümmung des Implantats eingerichtet ist und wenigstens einen Wirkabschnitt aufweist. Der Wirkabschnitt ist zur Aufnahme in der wenigstens einen Werkzeugaufnahmenut des Implantats eingerichtet. Vorteilhaft sind die Wirkabschnitte des Biegewerkzeugs auf die Werkzeugaufnahmenut oder die Werkzeugaufnahmenuten des Implantats formmäßig abgestimmt, so dass die Wirkabschnitte optimal in den Werkzeugaufnahmenuten aufgenommen sind und die Anlagefläche zwischen dem Implantat und dem Biegewerkzeug möglichst groß sind.

In Ausgestaltung der Erfindung ist der wenigstens eine Wirkabschnitt des Biegewerkzeugs beim intraoperativen Anpassen der Längskrümmung des Implantats in der wenigstens einen Werkzeugaufnahmenut des Implantats aufgenommen.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivansicht eine Vorderseite einer Ausführungsform eines erfindungsgemäßen Implantats zur Verbindung benachbarter Wirbel,
- Fig. 2: in schematischer Perspektivansicht eine Rückseite des Implantats nach Fig. 1,
- Fig. 3: in schematischer Seitenansicht das Implantat nach den Fig. 1 und 2, und
- Fig. 4: in schematischer Perspektivansicht eine Ausführungsform eines erfindungsgemäßen medizinischen Systems mit einem Biegewerkzeug und dem Implantat nach den Fig. 1 bis 3.

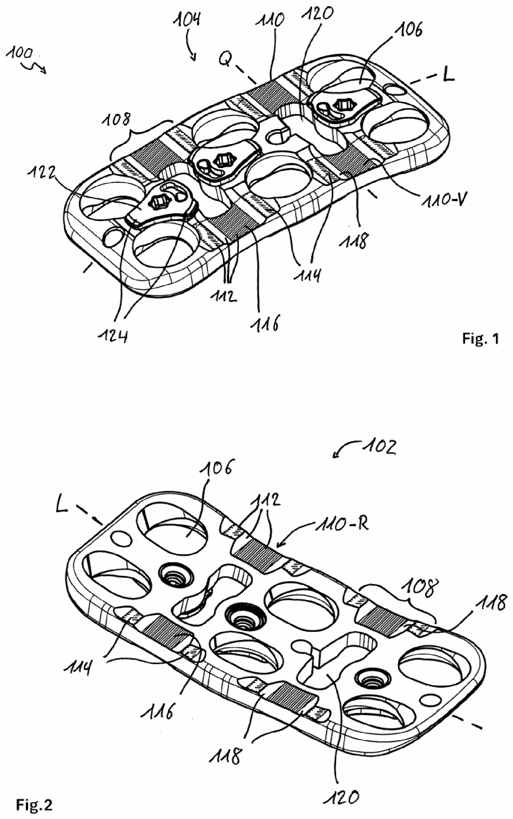

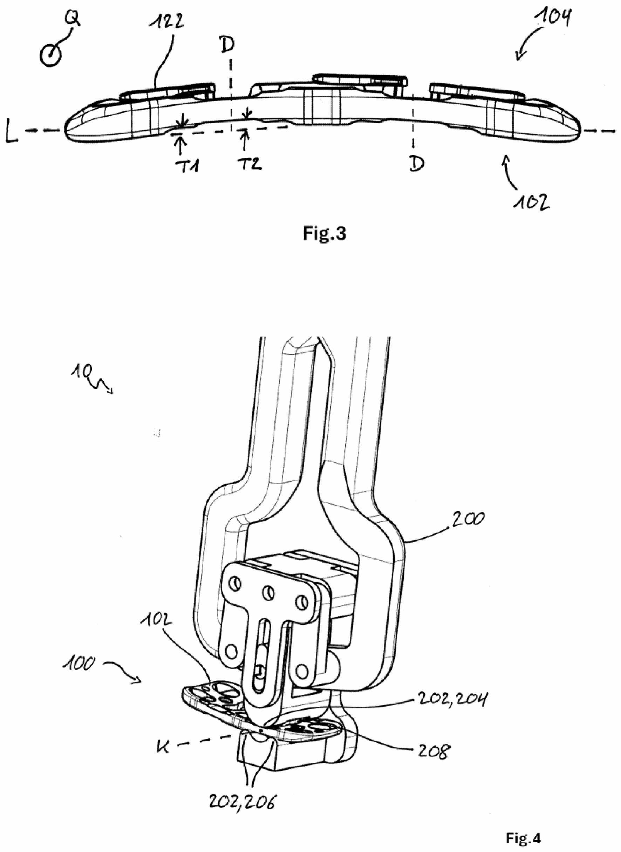

In Fig. 4 ist ein medizinisches System 10 mit einem Implantat 100 und einem Biegewerkzeug 200 gezeigt, wobei das Biegewerkzeug 200 zur Deformation des Implantats 100 dient. Das Implantat 100 ist im Detail in den Fig. 1 bis 3 gezeigt. Das Implantat 100 ist zur Verbindung von drei benachbarten Halswirbeln vorgesehen und weist eine Rückseite 102 (Fig. 2) und eine der Rückseite 102 in Dickenrichtung D gegenüberliegende Vorderseite 104 (Fig. 1) auf. Im implantierten Zustand ist die Rückseite 102 den Wirbeln zugewandt und die Vorderseite 104 den Wirbeln abgewandt. In der Regel wird das Implantat 100 ventral mit den Wirbeln der Halswirbelsäule verbunden. Die Vorderseite 104 weist dann in Blickrichtung des Patienten.

Das Implantat 100 umfasst bei der gezeigten Ausführungsform sechs Durchgangsöffnungen 106, von denen jeweils zwei Durchgangsöffnungen 106 einem der Wirbel zugeordnet sind. Entlang einer Längsachse L des Implantats 100 sind drei Durchgangsöffnungen 106 und entlang einer Querachse B zwei Durchgangsöffnungen 106 angeordnet. Zur Befestigung des Implantats 100 an den Wirbeln sind in den Durchgangsöffnungen 106 Befestigungsschrauben aufnehmbar, die jeweils paarweise in den Wirbeln verschraubt werden und das Implantat 100 mit den Wirbeln und damit die Wirbel untereinander verbinden. Das Implantat 100 ist vorliegend als dynamisches Implantat ausgebildet, womit gemeint ist, dass die Befestigungsschrauben in den Durchgangsöffnungen 106 um die Längsachse L und die Querachse B drehbeweglich und entlang der Längserstreckung der Durchgangsöffnungen 106 längsbeweglich gelagert sind und dadurch kleine Bewegungen der Wirbel relativ zueinander zulassen.

Bevor das Implantat 100 mit den Wirbeln verschraubt wird, wird es zwecks Anpassung an die Anatomie des betreffenden Patienten unter Verwendung des Biegewerkzeugs 200 plastisch verformt. Konkret wird ein Krümmungsradius entlang der Längsachse L lokal verändert, so dass das Implantat 100 einer ventralen Außenkontur der Wirbel folgt. Das Implantat 100 umfasst zwei Biegeabschnitte 108, in denen die Deformation im Wesentlichen erfolgt. Die Biegeabschnitte 108 sind entlang der Längsachse L hintereinander angeordnet und ermöglichen eine Veränderung der Längskrümmung an zwei Stellen. Jeder Biegeabschnitt 108 weist zwei Werkzeugaufnahmenuten 110 auf, eine hintere Werkzeugaufnahmenut 110-R auf der Rückseite 102 und eine vordere Werkzeugaufnahmenut 110-V auf der Vorderseite 104. Im Bereich der Werkzeugaufnahmenuten 110 ist der Querschnitt des Implantats 100 durch Wegnahme von Material in Dickenrichtung B reduziert und dadurch eine Biegesteifigkeit des Implantats 100 in diesem Bereich verringert. Hierdurch wird zum einen sichergestellt, dass die Verformung des Implantats im Wesentlichen in den Biegeabschnitten 108 erfolgt. Zum anderen wird dadurch die erforderliche Verformungskraft verringert, die durch das Biegewerkzeug 200 aufgebracht werden muss. Die vordere und die hintere Werkzeugaufnahmenut 110-V, 110-R sind in Bezug auf die Längsachse L auf gleicher Höhe vorgesehen. Auf diese Weise tragen die beiden Werkzeugaufnahmenuten 110 gemeinsam zu der Querschnittsverringerung bei.

Das Biegewerkzeug 200, das in Fig. 4 gezeigt ist, umfasst mehrere Wirkabschnitte 202, mit denen es mit dem Implantat 100 in Kontakt kommt und auf dieses unter Einleitung der Verformungskraft einwirkt. Das Biegewerkzeug 200 umfasst als Wirkabschnitte 202 eine Biegeform 204 und zwei Gegenhaltefortsätze 206, die der Biegeform 204 gegenüberliegend und in Bezug auf eine Biege- oder Krümmungsachse K des Implantats symmetrisch angeordnet sind. Das Implantat 100 wird im Gebrauch, das heißt beim Verformen, zwischen der Biegeform 204 einerseits und den Gegenhaltefortsätzen 206 andererseits angeordnet, so dass die Biegeform 204 in der vorderen Werkzeugaufnahmenut 110-V oder in der hinteren Werkzeugaufnahmenut 110-R aufgenommen ist und die Gegenhaltefortsätze 206 in der gegenüberliegenden hinteren Werkzeugaufnahmenut 110-R oder in der vorderen Werkzeugaufnahmenut 110-V aufgenommen sind. Durch Aufeinanderzubewegen der Biegeform 204 einerseits und der Gegenhaltefortsätze 206 andererseits wird das Implantat 100 durch eine Deformation im Biegeabschnitt 108 um die Biegeform 204 herum gekrümmt. Je weiter die Gegenhaltefortsätze 206 auf die Biegeform 204 zubewegt werden, desto stärker ist letztlich die erreichte Krümmung des Implantats 100. Das Biegewerkzeug 200 zentriert sich in einem als Fenster 120 bezeichneten Durchbruch im Implantat 100 entlang der Dickenrichtung D, indem ein zu dem Fenster 120 komplementär geformter Führungsvorsprung 208 des Biegewerkzeugs 200 in das Fenster 120 beim Deformieren des Implantats 100 eintaucht.

Die Werkzeugaufnahmenut 110 umfasst einen Nutgrund 112 mit einer ebenen Fläche, der zur Anlage gegen einen der Wirkabschnitte 202 eingerichtet ist. Der Wirkabschnitt 202 liegt entlang der Querachse B gegen den Nutgrund 112 an, wodurch eine vergleichsweise hohe Auflagefläche bereitgestellt ist. Wie insbesondere in den Fig. 1 bis 3 zu sehen ist, ist die Werkzeugaufnahmenut 110 mehrstufig ausgestaltet. Die Werkzeugaufnahmenut 110 umfasst zwei erste Nutabschnitte 114 und einen entlang der Längsachse L dazwischen angeordneten zweiten Nutabschnitt 116. Der erste Nutabschnitt 114 weist eine erste Nuttiefe T1 auf, die von einer Außenkontur des Implantats 100 entlang der Vorderseite 104 oder der Rückseite 102 gemessen ist. Der zweite Nutabschnitt 116 weist eine zweite Nuttiefe T2 auf, die größer als die erste Nuttiefe T1 ist, so dass in Bezug auf eine Außenseite des Implantats 100 der Nutgrund 112 im zweiten Nutabschnitt 116 tiefer gelegen ist als im ersten Nutabschnitt 114. Im Zusammenwirken mit dem Biegewerkzeug 200 ist der zweite Nutabschnitt 116 zur Aufnahme der Biegeform 204 vorgesehen, so dass die Biegeform 204 gegen den Nutgrund 112 des zweiten Nutabschnitts 116 anliegt. Die Gegenhaltefortsätze 206 sind dazu vorgesehen, gegen den Nutgrund 112 der beiden ersten Nutabschnitte 114 anzuliegen. Hierdurch ist ein Abstand zwischen den Gegenhaltefortsätzen 206 einerseits und der Biegeform 204 andererseits vergleichsweise gering.

Der zweite Nutabschnitt 116 ist in Richtung der Längsachse L gesehen länger als die beiden ersten Nutabschnitte 114, wodurch ein relativ großer Bereich bereitgestellt ist, in dem die Verformung des Implantats 100 erfolgen kann. Gleichzeitig ist eine Gesamterstreckung des Biegeabschnitts 108 entlang der Längsachse L, also die Längserstreckung beider erster Nutabschnitte und des zweiten Nutabschnitts gemeinsam, möglichst klein gehalten.

Die ersten Nutabschnitte 114 und der zweite Nutabschnitt 116 sind durch einen Übergangsabschnitt 118 miteinander verbunden, in dem der Nutgrund 112 vom zweiten Nutabschnitt 116 zum ersten Nutabschnitt 114 jeweils linear ansteigt. Insgesamt ist der Biegeabschnitt 108 in Richtung der Längsachse L gesehen somit als mehrere nebeneinander angeordnete und ineinander übergehende, ebene Flächen ausgestaltet. Durch den Übergangsabschnitt 118 ist die Werkzeugaufnahmenut 110 im Bereich zwischen den ersten Nutabschnitten 114 konkav ausgebildet, wobei in diesen Bereich die konvexe Biegeform 204 im Gebrauch eingreift.

Die Werkzeugaufnahmenuten 110 erstrecken sich nur teilweise über das Implantat 100 entlang der Querachse B, so dass in einem zwischen den Querenden liegenden Mittenbereich keine Werkzeugaufnahmenut 110 vorhanden ist. In dem Mittenbereich in Bezug auf die Biegeabschnitte 108 ist jeweils eines der Fenster 120 entlang der Dickenrichtung D vorgesehen. Somit ist eine Biegesteifigkeit des Implantats 100 im Biegeabschnitt 108 weiter herabgesetzt. Das Biegewerkzeug 200 ist bei der gezeigten Ausführungsform mit dem Führungsvorsprung 208 im Bereich der Biegeform 200 versehen, der im Gebrauch in das Fenster 120 eintaucht und eine Führung oder Ausrichtung der Wirkabschnitte 202 relativ zum Implantat 100 unterstützt oder bereitstellt.

Auf der Vorderseite 104 sind drei Verriegelungsmechanismen 122 in Form von Drehschiebern angeordnet, die zur Sicherung der in den Durchgangsöffnungen 106 aufgenommenen Befestigungsschrauben eingerichtet sind. Hierzu werden die Verriegelungsmechanismen 122 um eine Drehachse parallel zur Dickenrichtung D gedreht, so dass jeweils einer von zwei Verriegelungsarmen 124 jedes Verriegelungsmechanismus 122 über einen Schraubenkopf der Befestigungsschrauben geschwenkt wird.

Zur Anpassung der Längskrümmung ist das Implantat 100 zwischen den Wirkabschnitten 202 des Biegewerkzeugs 200 aufgenommen und eingeklemmt, siehe Fig. 4. Durch Betätigen des Biegewerkzeugs 200 werden die Biegeform 204 einerseits und die Gegenhaltefortsätze 206 andererseits aufeinander zubewegt. Dabei liegen die Gegenhaltefortsätze 206 gegen den Nutgrund 112 jeweils der ersten Nutabschnitte 114 und die Biegeform 204 gegen den Nutgrund 112 des zweiten Nutabschnitts 116 an. Die Gegenhaltefortsätze 206 zwingen das Implantat 100 zur Verformung um die Biegeform 204 herum, wobei das Implantat 100 im Wesentlichen im Bereich des Biegeabschnitts 108 plastisch deformiert wird, bis eine gewünschte Längskrümmung des Implantats 100 erreicht ist.

## Patentansprüche

1. Implantat (100) zur Verbindung benachbarter Wirbel, aufweisend
eine Vorderseite (104),
eine Rückseite (102), die der Vorderseite (104) in Dickenrichtung (D) des Implantats (100) gegenüberliegt,
wenigstens zwei Durchgangsöffnungen (106), die jeweils zwischen der Vorderseite (104) und der Rückseite (102) längserstreckt und zur Aufnahme einer Befestigungsschraube eingerichtet sind,
wenigstens einen Biegeabschnitt (108), der unter Einwirkung eines Biegewerkzeugs (200) auf das Implantat (100) plastisch deformierbar ist, um eine entlang einer Längsachse (L) des Implantats (100) erstreckte Längskrümmung des Implantats (100) intraoperativ anzupassen,
wobei der Biegeabschnitt (108) wenigstens eine Werkzeugaufnahmenut (110) aufweist, die in Dickenrichtung (D) in die Vorderseite (104) oder die Rückseite (102) eingesenkt und entlang einer Querachse (Q) des Implantats (100) längserstreckt ist, und
wobei die wenigstens eine Werkzeugaufnahmenut (110) zur Aufnahme eines Wirkabschnitts (202) des Biegewerkzeugs (200) eingerichtet ist,
**dadurch gekennzeichnet, dass** sich die Werkzeugaufnahmenut (110) nur über einen Teil der Breite des Implantats (100) erstreckt.

2. Implantat (100) nach Anspruch 1, **dadurch gekennzeichnet, dass** der Biegeabschnitt (108) eine vordere Werkzeugaufnahmenut (110-V) auf der Vorderseite (104) und eine hintere Werkzeugaufnahmenut (110-R) auf der Rückseite (102) umfasst, die einander in Bezug auf die Längsachse (L) zumindest teilweise überlappen.

3. Implantat (100) nach Anspruch 1 oder 2, **dadurch gekennzeichnet, dass** die Werkzeugaufnahmenut (110) einen Nutgrund (112) mit wenigstens einer ebenen Fläche umfasst.

4. Implantat (100) nach einem der vorhergehenden Ansprüche, **dadurch gekennzeichnet, dass** die Werkzeugaufnahmenut (110) mehrstufig ist, so dass die Werkzeugaufnahmenut (110) einen ersten Nutabschnitt (114) mit einer ersten Nuttiefe (T1) und einen zweiten Nutabschnitt (116) mit einer zweiten Nuttiefe (T2) umfasst, wobei die erste Nuttiefe (T1) geringer als die zweite Nuttiefe (T2) ist.

5. Implantat (100) nach Anspruch 4, wobei der zweite Nutabschnitt (116) in Richtung der Längsachse (L) gesehen länger ist als der erste Nutabschnitt (114).

6. Implantat (100) nach einem der Ansprüche 4 oder 5, **dadurch gekennzeichnet, dass** der erste Nutabschnitt (114) und der zweite Nutabschnitt (116) miteinander verbunden sind durch einen Übergangsabschnitt (118), in dem der Nutgrund (112) vom zweiten Nutabschnitt (116) zum ersten Nutabschnitt (114) ansteigt.

7. Implantat (100) nach einem der vorhergehenden Ansprüche, **dadurch gekennzeichnet, dass** wenigstens ein Verriegelungsmechanismus (122) zum Verriegeln der Befestigungsschrauben in den Durchgangsöffnungen (106) vorgesehen ist.

8. Medizinisches System (10) aufweisend
ein Implantat (100) nach einem der vorhergehenden Ansprüche und
ein Biegewerkzeug (200), das zum intraoperativen Anpassen einer Längskrümmung des Implantats (100) eingerichtet ist und wenigstens einen Wirkabschnitt (202) aufweist,
wobei der Wirkabschnitt (202) zur Aufnahme in der wenigstens einen Werkzeugaufnahmenut (110) des Implantats (100) eingerichtet ist.

9. Medizinisches System (10) nach Anspruch 8, wobei der wenigstens eine Wirkabschnitt (202) das Biegewerkzeugs (200) beim intraoperativen Anpassen der Längskrümmung des Implantats (100) in der wenigstens einen Werkzeugaufnahmenut (110) des Implantats (100) aufgenommen ist.

## Claims

1. Implant (100) for connecting adjacent vertebrae, comprising
a front side (104),
a rear side (102) which is opposite the front side (104) in the direction of thickness (D) of the implant (100), at least two through-holes (106) which in each case are elongate between the front side (104) and the rear side (102) and are configured to receive a fastening screw, at least one bending portion (108) which is able to be plastically deformed under the effect of a bending tool (200) acting on the implant (100) in order to intraoperatively adapt a longitudinal curvature of the implant (100) extending along a longitudinal axis (L) of the implant (100),
wherein the bending portion (108) has at least one tool receptacle groove (110) which is recessed in the direction of thickness (D) into the front side (104) or the rear side (102) and is elongate along a transverse axis (Q) of the implant (100), and
wherein the at least one tool receptacle groove (110) is configured to receive an effective portion (202) of the bending tool (200),
**characterized in that** the tool receptacle groove (110) extends only over part of the width of the implant (100).

2. Implant (100) according to Claim 1, **characterized in that** the bending portion (108) comprises a front tool receptacle groove (110-V) on the front side (104) and a rear tool receptacle groove (110-R) on the rear side (102), which at least partially overlap one another with respect to the longitudinal axis (L).

3. Implant (100) according to Claim 1 or 2, **characterized in that** the tool receptacle groove (110) comprises a groove base (112) having at least one flat surface.

4. Implant (100) according to any one of the preceding claims, **characterized in that** the tool receptacle groove (110) is in multiple stages such that the tool receptacle groove (110) comprises a first groove portion (114) with a first groove depth (T1) and a second groove portion (116) with a second groove depth (T2), the first groove depth (T1) being less than the second groove depth (T2).

5. Implant (100) according to Claim 4, wherein the second groove portion (116), when viewed in the direction of the longitudinal axis (L), is longer than the first groove portion (114).

6. Implant (100) according to one of Claims 4 or 5, **characterized in that** the first groove portion (114) and the second groove portion (116) are connected to one another by a transition portion (118) in which the groove base (112) ascends from the second groove portion (116) to the first groove portion (114).

7. Implant (100) according to one of the preceding claims, **characterized in that** at least one locking mechanism (122) for locking the fastening screws in the through-holes (106) is provided.

8. Medical system (10), having
an implant (100) according to one of the preceding claims, and
a bending tool (200) which is configured for intraoperative adjustment of a longitudinal curvature of the implant (100) and has at least one effective portion (202),
wherein the effective portion (202) is configured for being received in the at least one tool receptacle groove (110) of the implant (100).

9. Medical system (10) according to Claim 8, wherein the at least one effective portion (202) of the bending tool (200) is received in the at least one tool receptacle groove (110) of the implant (100) during the intraoperative adjustment of the longitudinal curvature of the implant (100).

## Revendications

1. Implant (100) destiné à relier des vertèbres adjacentes, présentant
une face avant (104),
une face arrière (102) qui est opposée à la face avant (104) de l'implant (100) dans la direction de l'épaisseur (D),
au moins deux ouvertures de passage (106), qui s'étendent chacune longitudinalement entre la face avant (104) et la face arrière (102) et qui sont conçues pour recevoir une vis de fixation,
au moins une section de flexion (108), qui est plastiquement déformable sous l'action d'un outil de flexion (200) sur l'implant (100), afin d'adapter de manière peropératoire une courbure longitudinale de l'implant (100) s'étendant le long d'un axe longitudinal (L) de l'implant (100),
dans lequel la section de flexion (108) présente au moins une rainure de réception d'outil (110), qui est creusée, dans la direction de l'épaisseur (D), dans la face avant (104) ou dans la face arrière (102) et qui s'étend longitudinalement le long d'un axe transversal (Q) de l'implant (100) et
dans lequel ladite au moins une rainure de réception d'outil (110) est conçue pour recevoir une section efficace (202) de l'outil de flexion (200), **caractérisé en ce que** la rainure de réception d'outil (110) ne s'étend que sur une partie de la largeur de l'implant (100).

2. Implant (100) selon la revendication 1, **caractérisé en ce que** la section de flexion (108) comprend une rainure de réception d'outil avant (110-V) sur la face avant (104) et une rainure de réception d'outil arrière (110-R) sur la face arrière (102), qui se chevauchent au moins partiellement par rapport à l'axe longitudinal (L).

3. Implant (100) selon la revendication 1 ou 2, **caractérisé en ce que** la rainure de réception d'outil (110) comprend un fond de rainure (112) présentant au moins une surface plane.

4. Implant (100) selon l'une des revendications précédentes, **caractérisé en ce que** la rainure de réception d'outil (110) est à plusieurs étages de telle sorte que la rainure de réception d'outil (110) comprend une première section de rainure (114) présentant une première profondeur de rainure (T1) et une deuxième section de rainure (116) présentant une deuxième profondeur de rainure (T2), la première profondeur de rainure (T1) étant inférieure à la deuxième profondeur de rainure (T2).

5. Implant (100) selon la revendication 4, dans lequel la deuxième section de rainure (116), vue dans la direction de l'axe longitudinal (L), est plus longue que la première section de rainure (114).

6. Implant (100) selon l'une des revendications 4 ou 5, **caractérisé en ce que** la première section de rainure (114) et la deuxième section de rainure (116) sont reliées l'une à l'autre par une section de transition (118), dans laquelle le fond de rainure (112) s'élève à partir de la deuxième section de rainure (116) vers la première section de rainure (114).

7. Implant (100) selon l'une des revendications précédentes, **caractérisé en ce qu'**au moins un mécanisme de verrouillage (122), destiné à verrouiller les vis de fixation dans les ouvertures de passage (106), est prévu.

8. Système médical (10) présentant
un implant (100) selon l'une des revendications précédentes et
un outil de flexion (200), qui est conçu pour l'adaptation peropératoire d'une courbure longitudinale de l'implant (100) et qui présente au moins une section efficace (202),
dans lequel la section efficace (202) est conçue pour être logée dans ladite au moins une rainure de réception d'outil (110) de l'implant (100).

9. Système médical (10) selon la revendication 8, dans lequel ladite au moins une section efficace (202) de l'outil de flexion (200) est logée dans ladite au moins une rainure de réception d'outil (110) de l'implant (100) lors de l'adaptation peropératoire de la courbure longitudinale de l'implant (100).
